**Europäisches Patentamt**

**European Patent Office**

**Office européen des brevets**

(11) Numéro de publication: **0 175 609 B1**

(12) **FASCICULE DE BREVET EUROPEEN**

(45) Date de publication de fascicule du brevet: **11.03.92**   (51) Int. Cl.⁵: **A61K 47/00, A61K 31/245**

(21) Numéro de dépôt: **85401721.7**

(22) Date de dépôt: **05.09.85**

(54) **Composition anesthésique percutanée pour usage topique et procédé d'application.**

(30) Priorité: **08.09.84 GB 8422759**

(43) Date de publication de la demande:
**26.03.86 Bulletin 86/13**

(45) Mention de la délivrance du brevet:
**11.03.92 Bulletin 92/11**

(84) Etats contractants désignés:
**AT BE CH DE FR GB IT LI LU NL SE**

(56) Documents cités:
**EP-A- 0 002 425**
**DE-A- 2 413 143**
**DE-A- 3 234 350**
**US-A- 3 337 406**

**"Pharmazeutische Stoffliste" 7 Auflage, Seite 94**

(73) Titulaire: **SOCIETE D'ETUDES SCIENTIFIOUES ET INDUSTRIELLES DE L'ILE-DE-FRANCE**
**46, Boulevard de Latour-Maubourg**
**F-75340 Paris Cédex 07(FR)**

(72) Inventeur: **Woolfson, David**
**7 Laral Gardens Comté Antrim**
**Newtownnabbeys Irlande du Nord(GB)**
Inventeur: **McCafferty, Dermot**
**50 Casaeldona Rise**
**Belfast Irlande du Nord(GB)**

(74) Mandataire: **Gallochat, Alain**
**SOCIETE D'ETUDES SCIENTIFIOUES ET INDUSTRIELLES DE L'ILE DE FRANCE 46, Boulevard de Latour Maubourg**
**F-75340 Paris Cédex 07(FR)**

**Description**

L'invention concerne des compositions pharmaceutiques contenant de l'améthocaine base.

L'améthocaine base ou p-butylamino benzoate de 2-diméthylamino éthyle, est utilisée en préparations topiques pour produire une anesthésie de surface. Les précédentes tentatives d'obtention d'une anesthésie percutanée topique, dans laquelle l'agent anesthésique doit pénétrer le stratum corneum, se sont heurtées à de nombreuses difficultés :

1) de hautes concentrations en principe actif (pouvant atteindre 33 %) étaient nécessaires.

2) les compositions précédemment obtenues étaient inséparables d'effets secondaires inacceptables.

3) les compositions précédemment obtenues exigeaient l'emploi de pansements occlusifs.

4) les compositions précédemment obtenues nécessitaient une période d'attente prolongée avant de conférer l'anesthésie percutanée attendue.

L'améthocaine a été jusqu'ici dispersée, soit dans un mélange d'éthanol ou d'isopropanol (45 %), de glycérine (10 %) et d'eau (45 %), soit dans une pommade hydrophile (95 %) ou dans de la vaseline (95 %) ou encore dans le DMSO (diméthylsulfoxyde).

Les formulations contenant le mélange éthanol-(ou isopropanol)-glycérine-eauont tendance à être très "mobiles" de nature et, par conséquent, la délimitation du site à traiter est difficile. De plus ces solutions ne possèdent qu'une stabilité limitée.

Les autres formulations possèdent des inconvénients similaires.

Celles qui contiennent des pommades hydrophiles ou de la vaseline, retardent l'action de l'effet anesthésique, tandis que les compositions contenant du DMSO provoquent une douloureuse dislocation à la surface de l'épiderme.

Selon un aspect de l'invention, il est proposé une composition pour l'application locale, comprenant : de l'améthocaine, un agent gélifiant aqueux et de l'eau dans laquelle se forme le gel, l'améthocaine étant fortement et complètement maintenue en phase solide discontinue, de telle façon que dans ladite composition l'améthocaine soit protégée de l'hydrolyse pendant le stockage, mais que lorsque la composition est appliquée sur la peau, l'améthocaine fonde et devienne disponible pour l'absorption.

Selon un autre aspect de la présente invention, il est proposé une composition à usage d'anesthésique local percutané, comprenant un anesthésique, un agent gélifiant aqueux pour stabiliser l'anesthésique et de l'eau, la composition étant telle que, lors de l'emploi, l'anesthésique fonde et se disperse à travers le gel pour provoquer la pénétration cutanée.

L'invention comprend également le procédé, pour produire l'anesthésie locale percutanée à l'aide de la composition ci-dessus définie, en quantité efficace, sur une région intacte de la peau.

La présente invention propose une composition contenant de l'améthocaine base qui s'étale peu ou pas du tout après l'application sur la peau. La formulation est protégée par l'application d'un pansement non absorbant convenable (occlusif ou non) et laissée en contact avec la peau pendant une période minimum de 20 minutes. Puis le pansement et l'application sont retirés avant de procéder à l'acte chirurgical. La composition produit un effet anesthésique profond, suffisamment pénétrant pour bloquer les récepteurs nociceptifs sous-jacents (récepteurs de la douleur) , procurant ainsi la possibilité d'actes indolores : prélèvements de greffes épidermiques dans toute leur épaisseur, pénétration de la peau par des aiguilles à injection (par exemple, prise de sang) et autres procédés chirurgicaux mineurs.

La composition évite l'usage d'infiltrations anesthésiques locales dans de telles circonstances, et peut être aisément appliquée par un personnel non médical.

Selon la présente invention, la composition contenant de l'améthocaine pour l'anesthésie locale percutanée, peut comprendre 1 à 7 %, et de préférence 4 %, d'améthocaine dispersée dans un gel aqueux, tel que 0,5 à 2 % de carbomère ou 3 à 10 % de méthyl cellulose et 81 à 94,5 % d'eau ; ces pourcentages sont exprimés en poids, par rapport au poids total de la composition.

L'améthocaine en poudre est répartie dans le gel qui ne doit pas contenir d'autre phase lipophile. La structure cristalline (gros cristaux) dans un support de gel visqueux, stabilise l'améthocaine en retardant sa dissolution et donc l'hydrolyse alcaline de son groupement ester, sous réserve que l'on n'excède pas une température de 30 ° C. L'améthocaine fond aux environs de 41 ° C, mais dans la composition, son point de fusion est abaissé à 30-32° C.

La température cutanée est habituellement aux environs de 32° C. Quand la composition est appliquée à la surface de la peau, les gros cristaux d'améthocaine fondent lentement et se dispersent sous forme de minuscules gouttelettes huileuses, dans le gel. L'améthocaine est ainsi présente sous forme moléculaire, et sous réserve qu'aucune autre phase lipophile ne soit présente, ces gouttelettes huileuses sont hautement pénétrantes dans la peau et dans ses sous-structures.

La présence de phases lipophiles étrangères obligerait à augmenter les concentrations en améthocaine pour maintenr une efficacité thérapeutique similaire.

Il n'est pas nécessaire d'inclure un conservateur dans la composition, en raison de la propriété antimicrobienne de l'améthocaine.

La composition peut être préparée par des moyens techniques conventionnels. Par exemple, l'améthocaine base peut être ajoutée à un gel formé de carbomère ou de méthyl cellulose, et soigneusement mélangée. Alternativement, on peut former le sel de sodium du carbomère, selon les instructions du fabricant. Dans ces conditions, la composition peut être préparée comme précédemment, ou bien, l'améthocaine peut être ajoutée et dispersée dans l'eau, avant l'addition du sel de sodium du carbopol, le tout étant soigneusement mélangé jusqu'à formation du gel.

Les compositions selon l'invention sont aussi facilement étalées que retirées de la peau, n'y laissant pas de traces grasses, en raison de la base aqueuse du produit.

Les formulations suivantes sont données à titre d'exemples préférentiels, en accord avec la présente invention :

Exemple 1

On mélange, comme précédemment décrit, les ingrédients suivants :

| Améthocaine | 4 % |
|---|---|
| Carbopol sodique 934 | 1,2 % |
| Eau | 94,8 % |

Exemple 2

| Améthocaine | 4 % |
|---|---|
| Méthylcellulose 450 | 7 % |
| Eau | 89 % |

Les compositions selon l'invention sont appliquées sur la peau intacte en couche épaisse, protégée par un pansement non absorbant. La composition est laissée en place pendant une période minimum de 20 minutes, pouvant aller jusqu'à une heure, mais se situant de préférence autour de 30 minutes, après quoi le pansement et la composition sont retirés. A ce moment, selon les variations individuelles, l'anesthésie complète soit est installée, soit nécessite une attente complémentaire (jusqu'à 50 minutes) pour être pleinement développée. L'effet anesthésique persiste pendant 2 à 8 heures, en fonction également des variations individuelles.

**Revendications**

1. Composition anesthésique locale à usage topique constituée uniquement d'améthocaine base, d'un agent gélifiant aqueux et d'eau.

2. Composition selon la revendication 1, comprenant 1 à 7% en poids d'améthocaine par rapport au poids total de la composition.

3. Composition selon la revendication 1, comprenant 0,5 à 10% en poids d'un agent gélifiant aqueux, par rapport au poids total de la composition.

4. Composition selon la revendication 3, dans laquelle l'agent gélifiant aqueux est du carbomère.

5. Composition selon la revendication 4, comprenant 0,5 à 2% en poids de carbomère, par rapport au poids total de la composition.

6. Composition selon la revendication 3, dans laquelle l'agent gélifiant aqueux est la méthylcellulose.

EP 0 175 609 B1

**7.** Composition selon la revendication 6, comprenant 3 à 10 % en poids de méthylcellulose, par rapport au poids total de la composition.

**8.** Composition suivant la revendication 1, comprenant 81 à 94,5% en poids d'eau par rapport au poids total de la composition.

**9.** Procédé de préparation d'un composition selon l'une quelconque des revendications 1 à 8, pour application topique, qui consiste à mélanger de l'améthocaine, un agent gélifiant aqueux et de l'eau dans laquelle se forme le gel, l'améthocaine étant totalement et fortement maintenue en phase solide discontinue de telle façon que dans ladite composition l'améthocaine soit protégée de l'hydrolyse pendant le stockage, mais qu'elle fonde et devienne disponible pour l'absorption lorsque la composition est appliquée sur la peau.

**Claims**

**1.** A local anaesthetic composition for topical application, comprising exclusively amethocaine free base, an aqueous gelling agent and water.

**2.** A composition according to claim 1, comprising 1 to 7 % of amethocaine by weight based on the total weight of the composition.

**3.** A composition according to claim 1, comprising 0.5 to 10 % of an aqueous gelling agent by weight based on the total weight of the composition.

**4.** A composition according to claim 3, in which the aqueous gelling agent is a carbomer.

**5.** A composition according to claim 4, comprising 0.5 to 2 % of carbomer by weight based on the total weight of the composition.

**6.** A composition according to claim 3, in which the aqueous gelling agent is methylcellulose.

**7.** A composition according to claim 6, comprising 3 to 10 % of methylcellulose by weight based on the total weight of the composition.

**8.** A composition according to claim 1, comprising 81 to 94.5 % of water by weight based on the total weight of the composition.

**9.** A process for preparing a composition for topical application, according to any of claims 1 to 8, which consists in mixing amethocaine, an aqueous gelling agent and water in which the gel is formed, the amethocaine being completely retained as a discontinuous solid phase whereby the amethocaine is protected from hydrolysis during storage but melts and is made available for sorption when the composition is applied to the skin.

**Patentansprüche**

**1.** Topische, lokalanästhetische Zusammensetzung, gekennzeichnet lediglich durch Amethocain als freie Base, ein wässriges Gelierungsmittel und Wasser.

**2.** Zusammensetzung nach Anspruch 1, gekennzeichnet durch 1 bis 7 Gew.- % Amethocain, bezogen auf das Gesamtgewicht der Zusammensetzung.

**3.** Zusammensetzung nach Anspruch 1, gekennzeichnet durch 0,5 bis 10 Gew.-% eines wässrigen Gelierungsmittels, bezogen auf das Gesamtgewicht der Zusammensetzung.

**4.** Zusammensetzung nach Anspruch 3, dadurch gekennzeichnet, daß das wässrige Gelierungsmittel ein Carbomer ist.

**5.** Zusammensetzung nach Anspruch 4, gekennzeichnet durch 0,5 bis 2 Gew.-% Carbomer, bezogen auf

4

das Gesamtgewicht der Zusammensetzung.

6. Zusammensetzung nach Anspruch 3, dadurch gekennzeichnet, daß das wässrige Gelierungsmittel Methylcellulose ist.

7. Zusammensetzung nach Anspruch 6, gekennzeichnet durch 3 bis 10 Gew.-% Methylcellulose, bezogen auf das Gesamtgewicht der Zusammensetzung.

8. Zusammensetzung nach Anspruch 1, gekennzeichnet durch 81 bis 94,5 Gew.-% Wasser, bezogen auf das Gesamtgewicht der Zusammensetzung.

9. Verfahren zur Herstellung einer Zusammensetzung nach einem der Ansprüche 1 bis 8 für die topische Anwendung, dadurch gekennzeichnet, daß man Amethocain, ein wässriges Gelierungsmittel und Wasser zuzammenmischt, wobei sich das Gel in dem Wasser ausbildet, und das Amethocain vollständig als diskontinuierliche feste Phase zurückgehalten wird, so daß es während der Lagerung vor Hydrolyse geschützt wird und während der Anwendung auf der Haut schmilzt und für die Adsorption zur Verfügung steht.